# EUROPEAN PATENT SPECIFICATION

(11) **EP 2 145 876 B1**
(45) Date of publication and mention of the grant of the patent: **04.07.2012**
(21) Application number: 09251778.8
(22) Date of filing: 10.07.2009
(51) Int. Cl.: C07C 249/04, C07C 251/44

(54) **Method for producing oxime**
Verfahren zur Herstellung von Oxim
Procédé de fabrication d'oxime

(30) Priority: 15.07.2008 JP 2008183467
(43) Date of publication of application: 20.01.2010
(73) Proprietor: Sumitomo Chemical Company, Limited, Tokyo 104-8260 (JP)
(72) Inventor: Oikawa, Miyuki, Ichihara-shi Chiba (JP); Kitamura, Masaru, Niihama-shi Ehime (JP)
(74) Representative: Duckworth, Timothy John

(56) References cited:
- EP-A- 0 735 017
- JP-A- 2007 001 952
- WU P ET AL: "A Novel Titanosilicate with MWW Structure: II. Catalytic Properties in the Selective Oxidation of Alkenes" JOURNAL OF CATALYSIS, ACADEMIC PRESS, DULUTH, MN, US, vol. 202, no. 2, 10 September 2001 (2001-09-10), pages 245-255, XP004432448 ISSN: 0021-9517

## Description

### Field of the Invention

The present invention relates to a method for producing an oxime by ammoximation reaction of a ketone. The oxime is useful as a starting material of amide or lactam.

### BACKGROUND OF THE INVENTION

Ammoximation reaction of a ketone with hydrogen peroxide and ammonia in a solvent in the presence of a titanosilicate has been known in the art(JP 2006-169168 A, JP 2007-1952 A and JP 2007-238541 A). However, hydrogen peroxide has not been always cost-effective. Hence, a method of using an organic peroxide as a recyclable peroxide has been studied but is not always satisfactory in that the selectivity or yield of the oxime is not stable for industrial production.

### SUMMARY OF THE INVENTION

The present invention provides a method for producing an oxime, which comprises reacting ketone with an organic peroxide, and ammonia in a solvent in the presence of titanosilicate of a temperature of from 50 to 200°C, wherein the ketone and ammonia are fed into a reactor containing the solvent, titanosilicate and organic peroxide, and wherein the solvent is acetonitrile, propionitrile, butyronitrile, isobutyronitrile, trimethylacetonitrile, valeronitrile, isovaleronitrile and/or benzonitrile; or the alcohol is methyl alcohol, ethyl alcohol, n-propyl alcohol, n-butyl alcohol, s-butyl alcohol, t-butyl alcohol and/or t-amyl alcohol.

According to the present invention, an oxime can be produced with stable selectivity and yield.

### DESCRIPTION OF THE PREFERRED EMBODIMENTS

The ketone that may be used in the present invention is typically an aliphatic ketone, an alicyclic ketone, unsaturated ketone, or an aromatic ketone. The ketones may be used alone or as a mixture of two or more of them. Examples of the aliphatic ketone include dialkyl ketones such as acetone, ethyl methyl ketone and isobutyl methyl ketone. Examples of the unsaturated ketone include alkyl alkenyl ketones such as mesityl oxide. Examples of the aromatic ketone include alkyl aryl ketones such as acetophenone; diaryl ketones such as benzophenone. Examples of the alicyclic ketone include cycloalkanones such as cyclopentanone, cyclohexanone, cyclooctanone and cyclododecanone. Examples of the unsaturated ketone also include cycloalkenones such as cyclopentenone and cyclohexenone. Among these ketones, cycloalkanones are preferably used in the present invention.

The ketone may be obtained by oxidation of an alkane, oxidation (dehydrogenation) of a secondary alcohol, or hydration and oxidation (dehydrogenation) of an alkene.

Ammonia may be used in the form of a gas, liquid or solution in an organic solvent. The amount of ammonia is preferably adjusted so that the concentration of ammonia in the liquid phase of the reaction mixture becomes 1% by weight or more. By adjusting the concentration of ammonia in the liquid phase of the reaction mixture to the predetermined value or more, the conversion rate of a ketone and the selectivity of an oxime can be increased, and thus the yield of an oxime as the objective product can also be increased. The concentration of ammonia is preferably 1.5% by weight or more, and usually 10% by weigh or less, preferably 5% by weight or less. The amount of ammonia is usually 1 mol or more, and preferably 1.5 mol or more, per 1 mol of the ketone.

The solvent that may be used for the ammoximation reaction is acetonitrile, propionitrile, butyronitrile, isobutyronitrile, trimethylacetonitrile, valeronitrile, isovaleronitrile and benzonitrile; or is methyl alcohol, ethyl alcohol, n-propyl alcohol, n-butyl alcohol, s-butyl alcohol, t-butyl alcohol and/or t-amyl alcohol. If necessary, two or more kinds of them can be used. In the present invention, the water content in the liquid phase of the reaction mixture is preferably kept lower in view of the selectivity of the oxime.

The amount of the solvent is usually from 1 to 500 parts by weight, and preferably from 2 to 300 parts by weight, per 1 part by weight of the ketone.

The titanosilicate that may be used in the present invention typically is a titanosilicate that contains titanium, silicon and oxygen as elements constituting its framework, and the framework may be composed of titanium, silicon and oxygen. Alternatively, the titanosilicate may contain elements other than titanium, silicon and oxygen, for example, boron, aluminum, gallium, iron, and chromium as elements constituting the framework. Specific examples of the titanosilicate include Ti-MWW as a crystalline titanosilicate having an MWW structure, TS-1 as a crystalline titanosilicate having an MFI structure, Ti-MCM-41 as a noncrystalline titanosilicate having a mesopore structure and the like. "MWW" and "MFI" are structural codes of zeolite defined by the International Zeolite Association (IZA).

The amount of the titanosilicate is usually adjusted within a range from 0.1 to 10% by weight relative to the liquid phase of the reaction mixture.

Examples of the organic peroxide that may be used include organic hydroperoxides such as t-butyl hydroperoxide, cumene hydroperoxide, cyclohexyl hydroperoxide, diisopropylbenzene hydroperoxide, p-methane hydroperoxide and 1,1,3,3-tetramethylbutyl hydroperoxide; dialkyl peroxides such as t-butylcumyl peroxide, di-t-butyl peroxide, di-t-hexyl peroxide, dicumyl peroxide, α,α'-di(t-butylperoxy)diisopropylbenzene, 2,5-dimethyl-2,5-di(t-butylperoxy)hexane and 2,5-dimethyl-2,5-bis(t-butylperoxy)hexyne-3; peroxy esters such as cumyl peroxy neodecanoate, 1,1,3,3-tetramethylbutyl peroxy neodecanoate, t-hexyl peroxy neodecanoate, t-butyl peroxy neodecanoate, t-butyl peroxy neoheptanoate, t-hexyl peroxy valeate, t-butyl peroxy pivalate, 2,5-dimethyl-2,5-di(2-ethylhexanoylperoxy)hexane, 1,1,3,3-tetramethylbutyl peroxy-2-ethyl hexanoate, t-hexyl peroxy-2-ethyl hexanoate, t-butyl peroxy-2-ethyl hexanoate, t-butyl peroxy laurate, t-butyl peroxy-3,5,5-trimethyl hexanoate, t-hexyl peroxy isopropyl monocarbonate, t-butyl peroxy-2-ethylhexyl monocarbonate, 2,5-dimethyl-2,5-di(benzoylperoxy)hexane, t-butyl peroxy acetate, t-hexyl peroxy benzoate and t-butyl peroxy benzoate; diacyl peroxides such as disiobutyryl peroxide, di(3,5,5-trimethylhexanoyl)peroxide, dilauroyl peroxide, disuccinic acid peroxide, dibenzoyl peroxide and di(4-methylbenzoyl)peroxide; and peroxy dicarbonates such as diisopropyl peroxy dicarbonate, di-n-propyl peroxy dicarbonate, bis(4-t-butylcyclohexyl)peroxy dicarbonate, di-2-ethylhexyl peroxy dicarbonate and di-sec-butyl peroxy dicarbonate. Among these organic peroxides, hydroperoxides are preferred.

In the ammoxidation reaction of the present invention, the organic peroxides are converted to an alcohol and/or carboxylic acid, which can be collected by distillation, extraction or the like for reuse as the organic peroxide, hence the present process is desirable for saving cost.

The amount of the organic peroxide that may be used is usually from 0.5 to 20 moles, and preferably from 0.5 to 10 moles, per 1 mol of a ketone.

Next, the mode of the ammoximation reaction will be explained. In the present invention, first, a solvent, a titanosilicate and an organic peroxide are introduced to a reactor. There is no particular limitation with respect to the order of introduction of them. Then, ketone and ammonia are typically fed to the reactor in which the titanosilicate is suspended by stirring. The ketone and ammonia are typically fed simultaneously as a ketone feed and an ammonia feed, which is referred to as "co-feed" in this specification, or as a mixture thereof. Preferably, a portion or ammonia may be preliminarily charged or fed together with the organic peroxide, and then the ketone and the remaining ammonia may be fed to the reactor. Alternatively, the organic peroxide may be preliminarily fed to the reactor and then the ketone, ammonia, and additional organic peroxide may be fed together.

The ammoximation reaction may be carried out batchwise or continuously. Particularly preferred is a continuous reaction process which comprises withdrawing a liquid phase of the resulting reaction mixture containing the product, and feeding reactants, typically ketone and ammonia, simultaneously in view of productivity and operability.

For example, a continuous reaction is preferably carried out by preparing a reaction mixture in which titanosilicate is suspended in a reactor, feeding reaction starting materials such as ketone and ammonia to the reactor, and withdrawing a liquid phase of the reaction mixture from the reactor through a filter.
Preferably employed is a reactor lined with glass or made of stainless steel for preventing of decomposition of the organic peroxide.

The temperature of the ammoximation reaction is from 50 to 200°C, and preferably from 80 to 150°C. The reaction pressure may be normal pressure, and is usually from 0.2 to 1 MPa absolute pressure, and preferably from 0.2 to 0.5 MPa, so as to readily dissolve ammonia in the liquid phase of the reaction mixture. The reaction pressure may be adjusted by using an inert gas such as nitrogen or helium.

The post-treatment operation of the resulting reaction mixture is appropriately selected. For example, an oxime can be separated by separating a titanosilicate from the reaction mixture through filtration or decantation and distillating a liquid phase.

### EXAMPLES

The present invention will be explained by way of the following examples and comparative examples. In the following examples, the liquid phase of the reaction mixture was analyzed by gas chromatography, and the conversion rate of cyclohexane as well as the selectivity and yield of cyclohexanone oxime were calculated based on the results of the analysis.

### Example 1

In a 1 L autoclave (reactor), 158.7 g of an acetonitrile solution containing 2.6% by weight of ammonia, 7.6 g of a cumene solution containing 80% by weight of cumene hydroperoxide and 5.0 g of Ti-MWW (prepared by the same manner as described in Chemistry Letters, 2000, pp. 774-775) were charged and a vapor phase portion in the reactor was replaced by nitrogen. After the reactor was sealed, the temperature in the reactor was raised to 110°C under stirring. The pressure in the reactor was 0.5 MPa. Next, 10 g of an acetonitrile solution containing 4.7% by weight of cyclohexanone, and 115 g of an acetonitrile solution containing 3.9% by weight of ammonia each were fed (co-fed) to the reactor over 1 hour. The concentration of ammonia in the liquid phase of the reaction mixture changed within a range from 1.0 to 2.6% by weight relative to the liquid phase.

After the co-feeding, the liquid phase of the reaction mixture was withdrawn and analyzed by gas chromatography. The conversion rate of cyclohexanone was found 73.3%, the selectivity to cyclohexanone oxime was 71.2% and the yield of cyclohexanone oxime was 52.2%. Selectivity to cyclohexanoneimine (a compound produced by imination of cyclohexanone) and impurities derived from the imine, based on the consumed cyclohexanone, was 21.1%.

### Example 2

In a 1 L autoclave (reactor), 158.7 g of an acetonitrile solution containing 2.6% by weight of ammonia, 5.5 g of an n-decane solution containing 65% by weight of t-butyl hydroperoxide and 5.0 g of Ti-MWW (prepared by the same manner as described in Chemistry Letters, 2000, pp. 774-775) were charged, and a vapor phase portion in the reactor was replaced by nitrogen. After the reactor was sealed, the temperature in the reactor was raised to 110°C under stirring. The pressure in the reactor was 0.5 MPa. Next, 10 g of an acetonitrile solution containing 4.7% by weight of cyclohexanone, and 115 g of an acetonitrile solution containing 3.8% by weight of ammonia each were fed (co-fed) to the reactor over 1 hour. The concentration of ammonia in the liquid phase of the reaction mixture changed within a range from 1.0 to 2.6% by weight relative to the liquid phase.

After the co-feeding, the liquid phase of the reaction mixture was withdrawn and analyzed by gas chromatography. The conversion rate of cyclohexanone was found 99.9%, the selectivity to cyclohexanone oxime was 63.6% and the yield of cyclohexanone oxime was 63.5%. Selectivity to cyclohexanoneimine (a compound produced by imination of cyclohexanone) and impurities derived from the imine, based on the consumed cyclohexanone, was 36.4%.

### Example 3

In a 1 L autoclave (reactor), 158.8 g of an ethanol solution containing 4.3% by weight of ammonia, 7.6 g of a cumene solution containing 80% by weight of cumene hydroperoxide and 5.0 g of Ti-MWW (prepared by the same manner as described in Chemistry Letters, 2000, pp. 774-775) were charged and a vapor phase portion in the reactor was replaced by nitrogen. After the reactor was sealed, the temperature in the reactor was raised to 110°C under stirring. The pressure in the reactor was 0.5 MPa.
Next, 10 g of an ethanol solution containing 4.7% by weight of cyclohexanone, and 115 g of an ethanol solution containing 3.8% by weight of ammonia each were fed (co-fed) to the reactor over 1 hour. The concentration of ammonia in the liquid phase of the reaction mixture changed within a range from 2.5 to 4.3% by weight relative to the liquid phase.

After the co-feeding, the liquid phase of the reaction mixture was withdrawn and analyzed by gas chromatography. The conversion rate of cyclohexanone was found 96.6%, the selectivity to cyclohexanone oxime was 37.3% and the yield of cyclohexanone oxime was 36.1%. Selectivity to cyclohexanoneimine (a compound produced by imination of cyclohexanone) and impurities derived from the imine, based on the consumed cyclohexanone, was 55.8%.

### Example 4

In a 1 L autoclave (reactor), 159.4 g of an acetonitrile solution containing 2.3% by weight of ammonia, 7.6 g of a cumene solution containing 80% by weight of cumene hydroperoxide and 5.0 g of Ti-MWW (prepared by the same manner as described in Chemistry Letters, 2000, pp. 774-775) were charged and a vapor phase portion in the reactor was replaced by nitrogen. After the reactor was sealed, the temperature in the reactor was raised to 115°C under stirring. The pressure in the reactor was 0.5 MPa. Next, 10 g of an acetonitrile solution containing 4.7% by weight of cyclohexanone, and 115 g of an acetonitrile solution containing 3.8% by weight of ammonia and 1.0% by weight of cumene hydroperoxide each were fed (co-fed) to the reactor over 1 hour. The concentration of ammonia in the liquid phase of the reaction mixture changed within a range from 1.0 to 2.3% by weight relative to the liquid phase.

After the co-feeding, the liquid phase of the reaction mixture was withdrawn and analyzed by gas chromatography. The conversion rate of cyclohexanone was found 99.5%, the selectivity to cyclohexanone oxime was 83.9% and the yield of cyclohexanone oxime was 83.5%. Selectivity to cyclohexanoneimine (a compound produced by imination of cyclohexanone) and impurities derived from imine, based on the consumed cyclohexanone, was 16.0%.

### Comparative Example 1

In a 1 L autoclave (reactor), 273 g of an acetonitrile solution containing 1.8% by weight of ammonia, 3.8 g of a cumene solution containing 80% by weight of cumene hydroperoxide, 5.0 g of Ti-MWW (prepared by the same manner as described in Chemistry Letters, 2000, pp. 774-775) and 2.0 g of cyclohexanone were charged and a vapor phase portion in the reactor was replaced by nitrogen. After the reactor was sealed, the temperature in the reactor was raised to 90°C under stirring. The pressure in the reactor was 0.2 MPa.
Next, stirring was carried out at the same temperature under the same pressure for 2 hours. Next, the liquid phase of the reaction mixture was withdrawn and analyzed by gas chromatography. The conversion rate of cyclohexanone was found 37.9%, the selectivity to cyclohexanone oxime was 26.6% and the yield of cyclohexanone oxime was 10.1%. Selectivity to cyclohexanoneimine (a compound produced by imination of cyclohexanone) and impurities derived from the imine, based on the consumed cyclohexanone, was 18.1%.

### Comparative Example 2

In a 1 L autoclave (reactor), 256 g of an ethanol solution containing 2.1% by weight of ammonia, 3.9 g of a cumene solution containing 80% by weight of cumene hydroperoxide, 5.0 g of Ti-MWW (prepared by the same manner as described in Chemistry Letters, 2000, pp. 774-775) and 2.0 g of cyclohexanone were charged and a vapor phase portion in the reactor was replaced by nitrogen. After the reactor was sealed, the temperature in the reactor was raised to 90°C under stirring. At this time, the pressure in the reactor was 0.2 MPa. Next, stirring was carried out at the same temperature under the same pressure for 2 hours. Next, the liquid phase of the reaction mixture was withdrawn and analyzed by gas chromatography. The conversion rate of cyclohexanone was found 41.1%, selectivity to cyclohexanone oxime was 8.3% and the yield of cyclohexanone oxime was 3.4%. Selectivity of the cyclohexanoneimine (a compound produced by imination of cyclohexanone) and impurities derived from the imine, based on the consumed cyclohexanone, was 25.7%.

### Comparative Example 3

In a 1 L autoclave (reactor), 243 g of a t-butanol solution containing 7.4% by weight of ammonia (also containing 12.5% by weight of water), 3.8 g of a cumene solution containing 80% by weight of cumene hydroperoxide, 10.0 g of TS-1 (prepared by the same manner as described in JP 56-96720 A) and 2.0 g of cyclohexanone were charged and a vapor phase portion in the reactor was replaced by nitrogen. After the reactor was sealed, the temperature in the reactor was raised to 90°C under stirring. The pressure in the reactor was 0.2 MPa.
Next, stirring was carried out at the same temperature under the same pressure for 6 hours. Then, the liquid phase of the reaction mixture was withdrawn and analyzed by gas chromatography. The conversion rate of cyclohexanone was found 10.9%, the selectivity to cyclohexanone oxime was 3.2% and the yield of cyclohexanone oxime was 0.4%. Selectivity to cyclohexanoneimine (a compound produced by imination of cyclohexanone) and impurities derived from the imine, based on the consumed cyclohexanone, was 5.9%.

## Claims

1. A method for producing an oxime, which comprises reacting ketone with an organic peroxide, and ammonia in a solvent in the presence of titanosilicate at a temperature of from 50 to 200°C, wherein the ketone and ammonia are fed into a reactor containing the solvent, titanosilicate and organic peroxide, and wherein the solvent is acetonitrile, propionitrile, butyronitrile, isobutyronitrile, trimethylacetonitrile, valeronitrile, isovaleronitrile and/or benzonitrile; or methyl alcohol, ethyl alcohol, n-propyl alcohol, n-butyl alcohol, s-butyl alcohol, t-butyl alcohol and/or t-amyl alcohol.

2. The method according to claim 1, wherein the ketone is comprised of one or more of an aliphatic ketone, an alicyclic ketone, an unsaturated ketone, or an aromatic ketone.

3. The method according to claim 2, wherein the aliphatic ketone is a dialkyl ketone; or the alicyclic ketone is a cycloalkanone; or the unsaturated ketone is a cycloalkenone; or the aromatic ketone is an alkyl aryl ketone or a diaryl ketone.

4. The method according to claim 3, wherein the ketone is cycloalkanone.

5. The method according to any of the preceding claims, wherein the organic peroxide is an organic hydroperoxide, a dialkyl peroxide, a peroxy ester, a diacyl peroxide or a peroxy dicarbonate.

6. The method according to claim 5, wherein the organic peroxide is an organic hydroperoxide.

7. The method according to any of the preceding claims, wherein the amount of the organic peroxide that may be used is from 0.5 to 20 moles, per 1 mol of ketone.

8. The method according to any of the preceding claims, wherein the ketone, ammonia and an additional amount of organic peroxide are fed into a reactor containing the solvent, titanosilicate and a preliminary amount of organic peroxide.

9. The method according to any of the preceding claims, wherein the amount of solvent is from 1 to 500 parts by weight, per 1 part by weight of the ketone.

10. The method according to any of the preceding claims, wherein the titanosilicate is Ti-MWW, TS-I or Ti-MCM-41.

11. The method according to any of the preceding claims, wherein the amount of titanosilicate is in the range of from 0.1 to 10% by weight relative to the liquid phase of the reaction mixture.

12. The method according to any of the preceding claims, wherein the concentration of ammonia in the liquid phase of the resulting reaction mixture is adjusted to 1% by weight or more relative to the liquid phase.

13. The method according to any of the preceding claims, wherein the reaction pressure of the ammoximation reaction is from 0.2 to 1 Mpa.

## Patentansprüche

1. Ein Verfahren zur Herstellung eines Oxims, welches das Umsetzen von Keton mit einem organischen Peroxid und Ammoniak in einem Lösungsmittel in Gegenwart von Titanosilicat bei einer Temperatur von 50 bis 200°C umfasst, wobei das Keton und der Ammoniak in einen Reaktor gegeben werden, der das Lösungsmittel, Titanosilicat und organisches Peroxid enthält, und wobei das Lösungsmittel Acetonitril, Propionitril, Butyronitril, Isobutyronitril, Trimethylacetonitril, Valeronitril, Isovaleronitril und/oder Benzonitril; oder Methylalkohol, Ethylalkohol, n-Propylalkohol, n-Butylalkohol, s-Butylalkohol, t-Butylalkohol und/oder t-Amylalkohol ist.

2. Das Verfahren gemäß Anspruch 1, wobei das Keton eines oder mehrere aus einem aliphatischen Keton, einem alicyclischen Keton, einem ungesättigten Keton oder einem aromatischen Keton umfasst.

3. Das Verfahren gemäß Anspruch 2, wobei das aliphatische Keton ein Dialkylketon ist; oder das alicyclische Keton ein Cycloalkanon ist; oder das ungesättigte Keton ein Cycloalkenon ist; oder das aromatische Keton ein Alkylarylketon oder ein Diarylketon ist.

4. Das Verfahren gemäß Anspruch 3, wobei das Keton Cycloalkanon ist.

5. Das Verfahren gemäß einem der vorhergehenden Ansprüche, wobei das organische Peroxid ein organisches Hydroperoxid, ein Dialkylperoxid, ein Peroxyester, ein Diacylperoxid oder ein Peroxydicarbonat ist.

6. Das Verfahren gemäß Anspruch 5, wobei das organische Peroxid ein organisches Hydroperoxid ist.

7. Das Verfahren gemäß einem der vorhergehenden Ansprüche, wobei die Menge des organischen Peroxids, die verwendet werden kann, 0,5 bis 20 Mol, bezogen auf 1 Mol Keton, beträgt.

8. Das Verfahren gemäß einem der vorhergehenden Ansprüche, wobei das Keton, der Ammoniak und eine zusätzliche Menge an organischem Peroxid in einen Reaktor gegeben werden, der das Lösungsmittel, Titanosilicat und eine vorläufige Menge an organischem Peroxid enthält.

9. Das Verfahren gemäß einem der vorhergehenden Ansprüche, wobei die Menge an Lösungsmittel von 1 bis 500 Gewichtsteile, bezogen auf 1 Gewichtsteil des Ketons, beträgt.

10. Das Verfahren gemäß einem der vorhergehenden Ansprüche, wobei das Titanosilicat Ti-MWW, TS-1 oder Ti-MCM-41 ist.

11. Das Verfahren gemäß einem der vorhergehenden Ansprüche, wobei die Menge an Titanosilicat im Bereich von 0,1 bis 10 Gew.-%, bezogen auf die flüssige Phase des Reaktionsgemisches, beträgt.

12. Das Verfahren gemäß einem der vorhergehenden Ansprüche, wobei die Konzentration von Ammoniak in der flüssigen Phase des resultierenden Reaktionsgemisches auf 1 Gew.-% oder mehr, bezogen auf die flüssige Phase, eingestellt wird.

13. Das Verfahren gemäß einem der vorhergehenden Ansprüche, wobei der Reaktionsdruck der Ammoximierungsreaktion 0,2 bis 1 MPa beträgt.

## Revendications

1. Procédé de fabrication d'oxime, qui comprend la réaction d'une cétone avec un peroxyde organique et de l'ammoniaque dans un solvant en présence de titanosilicate à une température de 50 à 200 °C, dans lequel la cétone et l'ammoniaque sont introduits dans un réacteur contenant le solvant, le titanosilicate et le peroxyde organique, et dans lequel le solvant est de l'acétonitrile, du propionitrile, du butyronitrile, de l'isobutyronitrile, du triméthylacétonitrile, du valéronitrile, de l'isovaléronitrile et/ou du benzonitrile ; ou de l'alcool méthylique, de l'alcool éthylique, de l'alcool n-propylique, de l'alcool n-butylique, de l'alcool s-butylique, de l'alcool t-butylique et/ou de l'alcool t-amylique.

2. Procédé selon la revendication 1, dans lequel la cétone est constituée d'un ou plusieurs composés parmi une cétone aliphatique, une cétone alicyclique, une cétone insaturée et une cétone aromatique.

3. Procédé selon la revendication 2, dans lequel la cétone aliphatique est une dialkylcétone ; ou la cétone alicyclique est une cycloalcanone ; ou la cétone insaturée est une cycloalcénone ; ou la cétone aromatique est une alkylarylcétone ou une diarylcétone.

4. Procédé selon la revendication 3, dans lequel la cétone est une cycloalcanone.

5. Procédé selon l'une quelconque des revendications précédentes, dans lequel le peroxyde organique est un hydroperoxyde organique, un peroxyde de dialkyle, un peroxyester, un peroxyde de diacyle ou un peroxydicarbonate.

6. Procédé selon la revendication 5, dans lequel le peroxyde organique est un hydroperoxyde organique.

7. Procédé selon l'une quelconque des revendications précédentes, dans lequel la quantité de peroxyde organique qui peut être utilisée est de 0,5 à 20 moles par mole de cétone.

8. Procédé selon l'une quelconque des revendications précédentes, dans lequel la cétone, l'ammoniaque et une quantité supplémentaire de peroxyde organique sont introduits dans un réacteur contenant le solvant, le titanosilicate et une quantité préliminaire de peroxyde organique.

9. Procédé selon l'une quelconque des revendications précédentes, dans lequel la quantité de solvant est de 1 à 500 parties en poids par partie en poids de cétone.

10. Procédé selon l'une quelconque des revendications précédentes, dans lequel le titanosilicate est du Ti-MWW, du TS-1 ou du Ti-MCM-41.

11. Procédé selon l'une quelconque des revendications précédentes, dans lequel la quantité de titanosilicate est dans la plage de 0,1 à 10 % en poids par rapport à la phase liquide du mélange réactionnel.

12. Procédé selon l'une quelconque des revendications précédentes, dans lequel la concentration d'ammoniaque dans la phase liquide du mélange réactionnel résultant est ajustée à 1 % en poids ou plus par rapport à la phase liquide.

13. Procédé selon l'une quelconque des revendications précédentes, dans lequel la pression de réaction de la réaction d'ammoximation est de 0,2 à 1 MPa.
